# FASCICULE DE BREVET EUROPEEN

(11) **EP 2 078 533 B1**
(45) Date de publication et mention de la délivrance du brevet: **12.01.2011**
(21) Numéro de dépôt: 09290009.1
(22) Date de dépôt: 07.01.2009
(51) Int. Cl.: A61M 1/10

(54) **Prothèse cardiaque monobloc implantable**
Einteilige implantierbare Herzprothese
Implantable single-piece artificial heart

(30) Priorité: 14.01.2008 FR 0800184
(43) Date de publication de la demande: 15.07.2009
(73) Titulaire: CARMAT, 78140 Vélizy Villacoublay (FR)
(72) Inventeur: Grimmé, Marc, 75019 Paris (FR); Gourgues, Jean Elie, 78000 Versailles (FR); Carpentier, Alain, 75116 Paris (FR); Wartelle, Claude, 60270 Gouvieux (FR)
(74) Mandataire: Bonnetat, Christian

(56) Documents cités:
- WO-A-01/91828
- FR-A- 2 585 250
- US-A- 4 687 424
- US-A- 5 135 539

## Description

La présente invention concerne une prothèse cardiaque monobloc implantable.

Par le document US-5 135 539, on connaît déjà une prothèse cardiaque implantable dans la cavité péricardique d'un patient, ladite prothèse étant apte à remplacer les ventricules gauche et droit naturels dudit patient après ablation de ceux-ci et comportant :
- un corps rigide dans lequel sont agencés des ventricules gauche et droit artificiels, chacun de ces ventricules artificiels comprenant une membrane souple pulsatile :
   ■ qui est apte à battre sous l'action d'un fluide hydraulique, et
   ■ qui est disposée dans une cavité que ladite membrane partage de façon étanche en deux chambres dont l'une est destinée à la circulation du sang et dont l'autre est destinée audit fluide hydraulique, la chambre à sang du ventricule artificiel gauche étant destinée à être reliée à l'oreillette naturelle gauche et à l'aorte, alors que la chambre à sang du ventricule artificiel droit est destinée à être reliée à l'oreillette naturelle droite et à l'artère pulmonaire ;
- deux actionneurs hydrauliques reliés aux chambres à fluide hydraulique desdites cavités dudit corps rigide, pour alternativement y injecter et en expulser du fluide hydraulique et assurer des valeurs désirées de débits diastoliques et systoliques ; et
- un sac souple entourant, de façon ample et étanche, au moins une partie dudit corps rigide en enfermant lesdits actionneurs hydrauliques, ledit sac souple servant à la fois de réservoir de fluide hydraulique pour lesdits actionneurs hydrauliques et de chambre de compliance.

Dans cette prothèse cardiaque connue, chaque actionneur est associé à un ventricule et, pour respecter la physiologie, les deux actionneurs peuvent fonctionner indépendamment l'un de l'autre et notamment en synchronisme, c'est-à-dire que les deux ventricules peuvent être respectivement et simultanément en diastole ou en systole. Dans ce cas, il en résulte que ledit sac souple est le siège de déplacements importants, puisque la totalité du fluide nécessaire à l'animation des membranes pulsatiles droite et gauche est alternativement injectée, puis aspirée dans ledit sac souple. Si la capacité de chaque ventricule est de l'ordre de 75 cm³, les variations du volume du sac peuvent atteindre 150 cm³. De tels battements du sac de fortes amplitudes, d'une part, peuvent soulever des difficultés de logement de ladite prothèse dans la cavité péricardique et, d'autre part, provoquent une inflammation des tissus environnants, risquant de développer une capsule fibreuse épaisse apte à gêner les battements du sac et à pénaliser le fonctionnement de la prothèse.

Par ailleurs, un tel fonctionnement en synchronisme nécessite que les deux actionneurs soient capables des mêmes performances, ce qui est coûteux en énergie.

Pour remédier à ces inconvénients, on pourrait, comme suggéré par le document WO-O 191 828, supprimer l'un desdits actionneurs et faire fonctionner les ventricules en opposition de phase, l'un desdits ventricules étant en diastole pendant que l'autre est en systole. Ainsi, le fluide hydraulique est transféré d'un ventricule dans l'autre avec des battements très réduits du sac. De plus, une telle prothèse cardiaque est avantageuse en encombrement et en consommation d'énergie, puisqu'elle ne comporte qu'un seul actionneur. Cependant, elle présente l'inconvénient de réguler la durée d'admission d'un des ventricules sur l'éjection de l'autre, de sorte que les durées des diastoles sont obligatoirement égales aux durées des systoles, ce qui ne permet pas de respecter la physiologie. De plus, une telle prothèse cardiaque à actionneur unique présente le risque, en fonctionnement, soit d'aspirer les oreillettes et de mal remplir les ventricules, soit d'éjecter insuffisamment vite et de ne pas maintenir une pression correcte.

La présente invention a pour objet de remédier à ces inconvénients en perfectionnant la prothèse cardiaque à deux actionneurs rappelée ci-dessus.

A cette fin, selon l'invention, une telle prothèse cardiaque à deux actionneurs est remarquable en ce que :
- l'un desdits actionneurs est principal et est disposé entre les chambres à fluide hydraulique des deux ventricules artificiels, dont l'un est menant et l'autre est mené ;
- l'autre desdits actionneurs est auxiliaire et est disposé entre la chambre à fluide hydraulique dudit ventricule artificiel mené et ledit réservoir de fluide hydraulique constitué par ledit sac souple ;
- l'actionneur principal :
   ■ adresse, audit ventricule artificiel menant, des débits diastolique et systolique de valeurs respectives désirées pour ce ventricule artificiel menant, et
   ■ adresse, audit ventricule artificiel mené :
      * un débit systolique inverse dudit débit diastolique de valeur désirée pour ledit ventricule artificiel menant, et
      * un débit diastolique inverse dudit débit systolique de valeur désirée pour ledit ventricule artificiel menant ; et
- l'actionneur auxiliaire adresse, audit ventricule artificiel mené, des débits systolique et diastolique de correction pour corriger lesdits débits systolique et diastolique adressés par ledit actionneur principal audit ventricule artificiel mené et pour communiquer respectivement aux débits systolique et diastolique corrigés des valeurs désirées pour ledit ventricule artificiel mené.

Ainsi, grâce à la présente invention, les battements du sac étanche sont limités puisque, en fonctionnement, le fluide hydraulique est transféré d'un ventricule artificiel à l'autre. Cependant, grâce auxdits débits de correction engendrés par ledit actionneur auxiliaire, les durées des diastoles et des systoles ne sont plus obligatoirement égales.

Par ailleurs, l'actionneur auxiliaire peut être de moindre puissance que ledit actionneur principal, de sorte que la consommation en énergie de la prothèse conforme à la présente invention est inférieure à la consommation énergétique de la prothèse connue à deux actionneurs. De plus, l'actionneur auxiliaire peut alors présenter des dimensions inférieures à celles dudit actionneur principal.

De préférence, le ventricule artificiel menant correspond au ventricule artificiel droit, alors que le ventricule artificiel mené correspond au ventricule artificiel gauche. De plus, il est avantageux que lesdits actionneurs principal et auxiliaire soient du type moto-pompe volumétrique.

Pour des raisons de commodité de logement de la prothèse à l'intérieur de la cavité péricardique, il est également avantageux que ledit actionneur principal et ledit actionneur auxiliaire soient disposés au voisinage l'un de l'autre. Dans ce cas, pour profiter de l'anatomie, lesdits actionneurs principal et auxiliaire sont disposés au voisinage du ventricule artificiel gauche et ils communiquent alors en commun avec la chambre à fluide hydraulique de ce dernier ventricule, tandis que ledit actionneur principal est relié à la chambre à fluide hydraulique du ventricule artificiel droit par un conduit extérieur audit corps rigide.

Les figures du dessin annexé feront bien comprendre comment l'invention peut être réalisée. Sur ces figures, des références identiques désignent des éléments semblables.
La figure 1 montre schématiquement, en coupe, une prothèse cardiaque connue que la présente invention a pour but de perfectionner.
La figure 2 illustre schématiquement l'état de la prothèse cardiaque de la figure 1, à la fin d'une systole.
La figure 3 illustre schématiquement l'état de la prothèse cardiaque de la figure 1, à la fin d'une diastole.
La figure 4 montre schématiquement, en vue semblable aux figures 1 à 3, la prothèse cardiaque conforme à la présente invention.
La figure 5 est le schéma fonctionnel de la prothèse cardiaque de la figure 4.
La figure 6 illustre le fonctionnement de la prothèse cardiaque des figures 4 et 5 pour des débits diastolique et systolique normaux.
La figure 7 illustre le fonctionnement de la prothèse cardiaque des figures 4 et 5 pour des débits diastolique et systolique élevés.
La figure 8 est une vue en perspective d'un mode de réalisation pratique de la prothèse de l'invention, son enveloppe constituée d'un sac souple étanche étant supposée transparente.
La figure 9 est une vue de dessus de la prothèse de la figure 8.
La figure 10 est une vue en perspective de la prothèse des figures 8 et 9, après élimination dudit sac souple étanche et de la paroi ajourée enveloppante.
La figure 11 est une vue en perspective montrant les actionneurs hydrauliques et les couvercles des ventricules artificiels de la prothèse des figures 8 à 10.

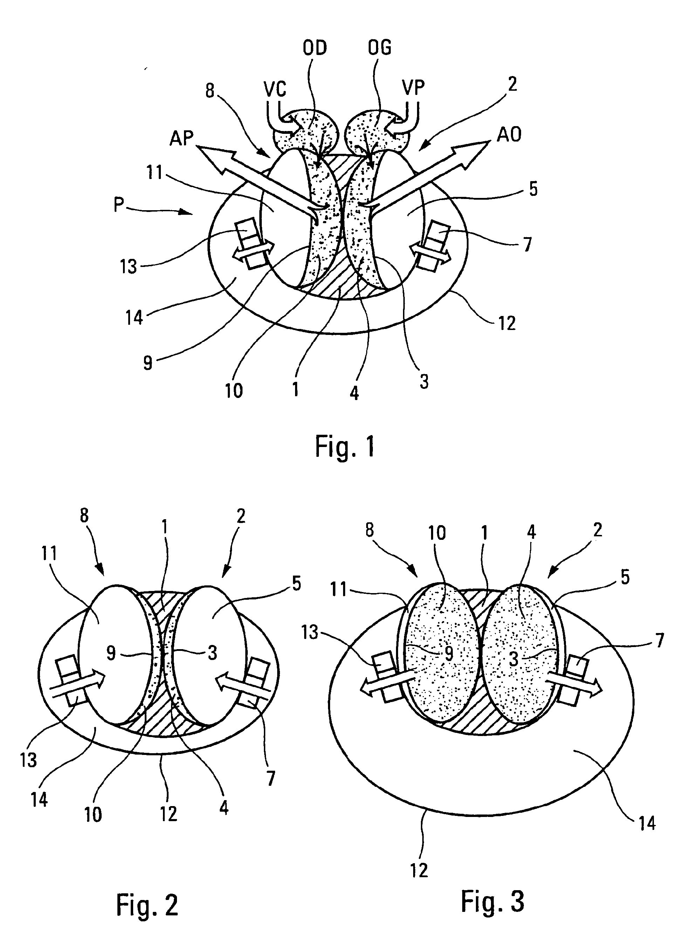

La prothèse connue P, représentée schématiquement sur la figure 1, est destinée à remplacer les ventricules gauche et droit naturels d'un coeur malade (non représentés), après ablation de ceux-ci. La prothèse P doit pouvoir être logée au moins sensiblement dans la partie de la cavité péricardique laissée libre par l'élimination desdits ventricules naturels.

Comme cela est représenté schématiquement par la figure 1, la prothèse P comporte :
- un corps rigide 1 dans lequel est agencé un ventricule gauche artificiel 2, comprenant une membrane souple 3 qui sépare de façon étanche ledit ventricule artificiel 2 en une chambre 4 pour la circulation du sang et en une chambre 5 pour un fluide hydraulique, ladite chambre à sang 4 étant destinée à être reliée, d'un côté, à l'oréillette naturelle gauche OG en communication avec les veines pulmonaires VP et, de l'autre côté, à l'aorte AO ;
- un actionneur hydraulique 7, par exemple du type moto-pompe volumétrique, en communication avec la chambre 5 à fluide hydraulique du ventricule gauche artificiel 2 ;
- un ventricule droit artificiel 8, agencé dans ledit corps rigide 1 et comprenant une membrane souple 9 qui sépare de façon étanche ledit ventricule artificiel 8 en une chambre 10 pour la circulation du sang et en une chambre 11 pour un fluide hydraulique, ladite chambre à sang 10 étant destinée à être reliée, d'un côté, à l'oreillette naturelle droite OD en communication avec les veines caves VC et, de l'autre côté, à l'artère pulmonaire AP ; et
- un actionneur hydraulique 13, par exemple également du type moto-pompe volumétrique, en communication avec la chambre 11 à fluide hydraulique du ventricule droit artificiel 8.

Par ailleurs, un sac souple 1 2 entoure, de façon ample et étanche, au moins une partie du corps rigide 1 en enfermant les actionneurs hydrauliques 7 et 13. Ce sac souple 12 forme un réservoir 14 pour le fluide hydraulique déplacé par lesdits actionneurs 7 et 13.

Dans la pompe P, chaque actionneur 7 et 13 est spécifiquement dédié à l'un des ventricules artificiels 2 ou 8, respectivement, de sorte que les deux actionneurs 7 et 13 sont de même puissance.

Lorsque, comme cela est illustré schématiquement par les figures 2 et 3, les actionneurs 7 et 13 sont commandés en phase, de façon que les systoles des ventricules artificiels 2 et 8 se produisent simultanément (figure 2) et que les diastoles desdits ventricules artificiels 2 et 8 soient également simultanées (figure 3), le volume du réservoir 14 constitué par le sac souple 12 varie dans de larges limites. En effet, dans le cas des systoles simultanées (figure 2), les deux chambres à fluide hydraulique 5 et 11 sont remplies de ce fluide, de sorte que le réservoir 14 contient peu de fluide hydraulique et que son volume est réduit. Au contraire, lorsque lesdits ventricules artificiels 2 et 8 sont dans l'état où leurs diastoles sont simultanées (figure 3), les deux chambres à fluide hydraulique 5 et 11 se vident, de sorte que le réservoir 14 est empli de fluide hydraulique et que son volume est important.

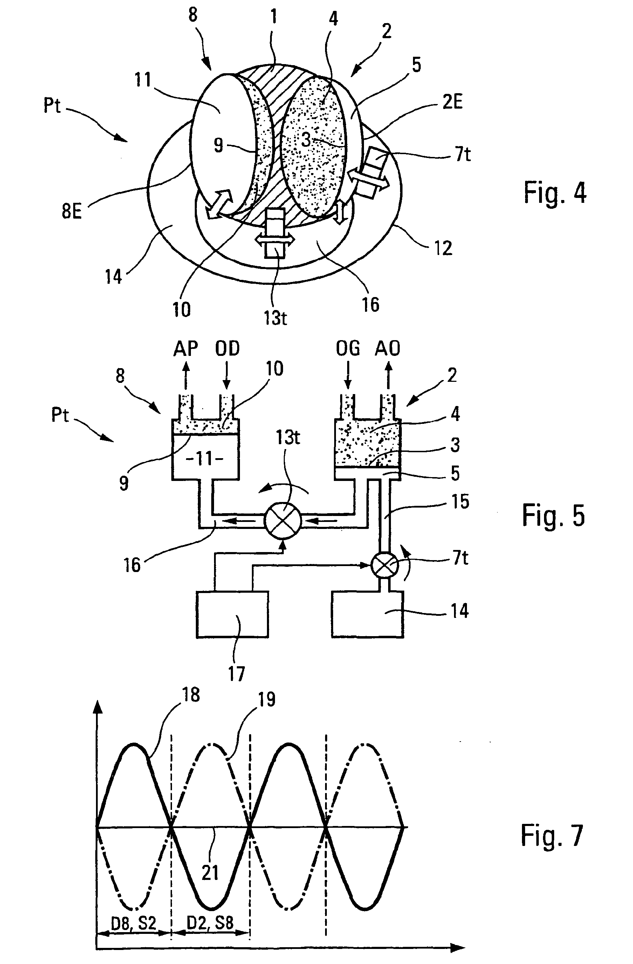

La prothèse Pt, conforme à la présente invention et représentée sur les figures 4, 5 et 8 à 11, permet d'éviter une telle variation importante du volume du réservoir 14, tout en nécessitant une puissance moindre pour son fonctionnement.

Cette prothèse Pt est identique à la prothèse P décrite ci-dessus en ce qui concerne les éléments 1 à 5, 8 à 12 et 14. En revanche :
- l'actionneur 7 est remplacé par un actionneur 7t de plus faible puissance ; et
- l'actionneur 13 est remplacé par un actionneur 13t de même puissance, mais disposé autrement. En effet, l'actionneur 13t n'est plus agencé (comme l'est l'actionneur 13) entre la chambre à fluide hydraulique 11 et le réservoir 14, mais entre les chambres à fluide hydraulique 5 et 11 des deux ventricules artificiels 2 et 8.

Ainsi, ledit actionneur 7t est de moindre puissance et peut être de plus faible encombrement que ledit actionneur 13t.

Le schéma fonctionnel de la prothèse Pt, qui est montré sur la figure 5 et dans lequel les ventricules artificiels 2 et 8 sont représentés sous la forme de cylindres dans lesquels se déplacent, respectivement, des pistons constitués par les membranes 3 et 9, permet de bien comprendre le fonctionnement de la prothèse Pt de la figure 4. On peut y voir que l'actionneur 7t est disposé dans une liaison 15 reliant le réservoir 14 et la chambre à fluide 5 du ventricule artificiel 2 et que l'actionneur 13t est disposé dans une liaison 16 reliant les deux chambres à fluide 5 et 11 des ventricules artificiels 2 et 8.

Un dispositif 17 commande le fonctionnement des actionneurs 7t et 13t, de façon que l'actionneur 13t joue un rôle principal, alors que l'actionneur 7t joue un rôle auxiliaire de correction de débit.

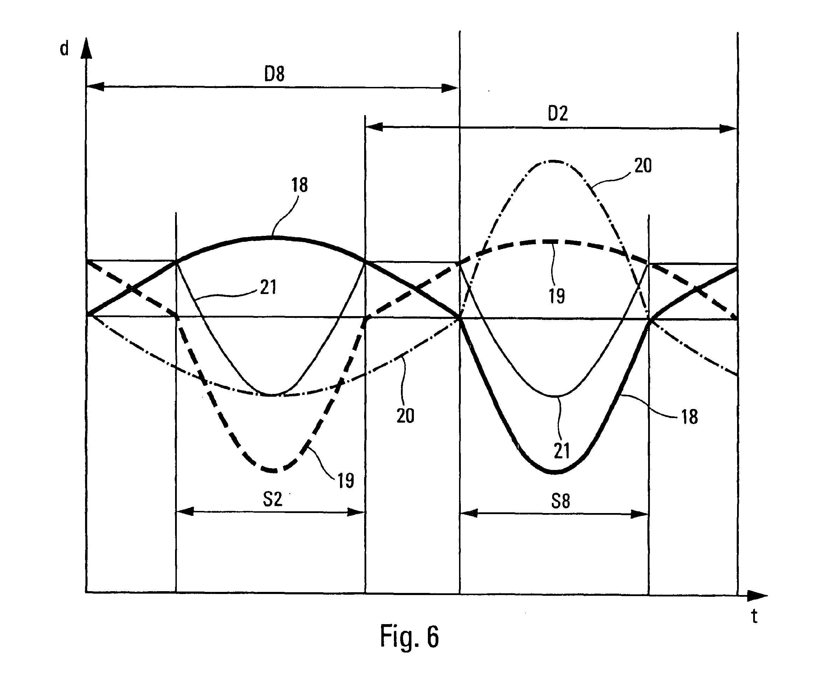

Le fonctionnement de la prothèse Pt est expliqué plus en détail ci-après en regard du chronogramme de la figure 6. Sur ce chronogramme, on a représenté différents débits de fluide hydraulique d (correspondant respectivement à des débits sanguins) en fonction du temps t. On y a notamment représenté (en trait plein) une courbe 18 correspondant à des débits de valeurs désirées pour l'actionneur principal 13t pendant les diastoles D8 et les systoles S8 du ventricule artificiel 8, ainsi que (en tirets) une courbe 19 correspondant à des débits de valeurs désirées pour l'actionneur auxiliaire 7t pendant les diastoles D2 et les systoles S2 du ventricule artificiel 2. On remarquera que, dans l'exemple représenté, les systoles S2 et S8 ont des durées plus faibles que les diastoles D2 et D8.

Le dispositif 17 commande l'actionneur principal 13t pour qu'il adresse, au ventricule artificiel 8, les débits diastolique et systolique désirés, représentés par la courbe 18. Le ventricule artificiel 8 délivre donc les volumes de sang désirés.

En revanche, du fait de l'existence de la liaison 16 entre les chambres à fluide hydraulique 5 et 11 des ventricules artificiels 2 et 8, l'actionneur principal 1 3t impose au ventricule artificiel 2 des débits systolique et diastolique représentés par la courbe 20 en trait mixte, tels que les débits systolique et diastolique dudit ventricule artificiel 2 sont respectivement les inverses des débits diastolique et systolique du ventricule artificiel 8 (courbe 18). Dans ce fonctionnement, le ventricule artificiel 8 est donc menant, alors que le ventricule artificiel 2 est mené.

Aussi, pour que le ventricule artificiel 2 puisse recevoir les débits désirés représentés par la courbe 19, le dispositif 17 commande l'actionneur auxiliaire 7t pour qu'il adresse, audit ventricule artificiel 2, des débits systolique et diastolique de correction (représentés par la courbe 21 sur la figure 6) aptes à corriger lesdits débits systolique et diastolique (courbe 20) imposés par l'actionneur principal 13t.

La figure 6 montre des formes de débits diastolique et systolique correspondant à des débits sanguins normaux moyens, par exemple de l'ordre de 5 litres par minute. Dans le cas où le débit sanguin est élevé, par exemple de l'ordre de 8 litres par minute, les formes de débit seraient plutôt comme représentés sur la figure 7. Dans ce cas, les courbes 18 et 19 ressembleraient à des sinusoïdes identiques en opposition de phase, de sorte qu'alors aucune correction ne serait nécessaire de la part de l'actionneur auxiliaire 7t (ce qui est représenté sur la figure 7 par le fait que la courbe 21 est confondue avec l'axe des sinusoïdes 18 et 19).

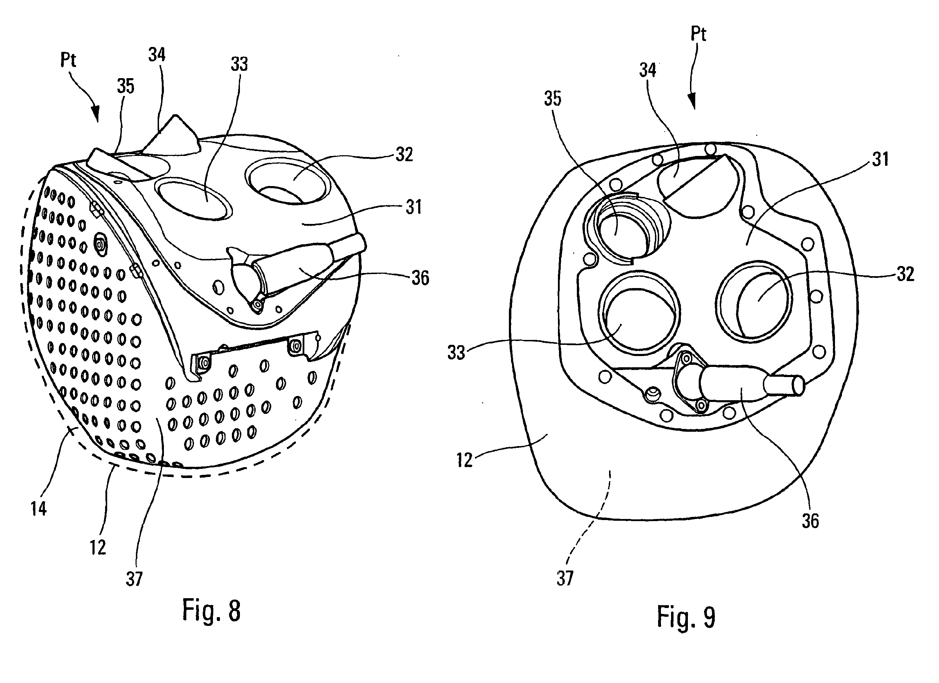

Dans le mode de réalisation pratique représenté par les figures 8 et 9, la prothèse Pt se présente sous la forme d'un volume de forme anatomique (correspondant à celle de la cavité péricardique) comportant une platine 31, sur laquelle sont ramenés un orifice 32 de raccord à l'oreillette naturelle gauche OG, un orifice 33 de raccord à l'oreillette naturelle droite OD, un orifice 34 de raccord à l'aorte AO et un orifice 35 de raccord à l'artère pulmonaire AP. De plus, sur ces figures, on a représenté la base 36 d'une connexion électrique avec l'extérieur de ladite prothèse Pt.

La prothèse cardiaque est enfermée de façon étanche dans le sac souple 12 (supposé transparent sur la figure 8) entourant ladite prothèse, de façon ample, et rempli du fluide hydraulique actionné par les actionneurs 7t et 13t, immergés dans ce fluide. Le sac souple 12 forme le réservoir 14 servant de bâche pour ce fluide hydraulique.

Le corps 1 et les actionneurs 7t et 13t sont enveloppés par une paroi rigide ajourée 37, faisant crépine et permettant la circulation du fluide hydraulique à l'intérieur du sac 12. La paroi perforée 37 évite audit sac souple d'être aspiré par les actionneurs 7t et 13t.

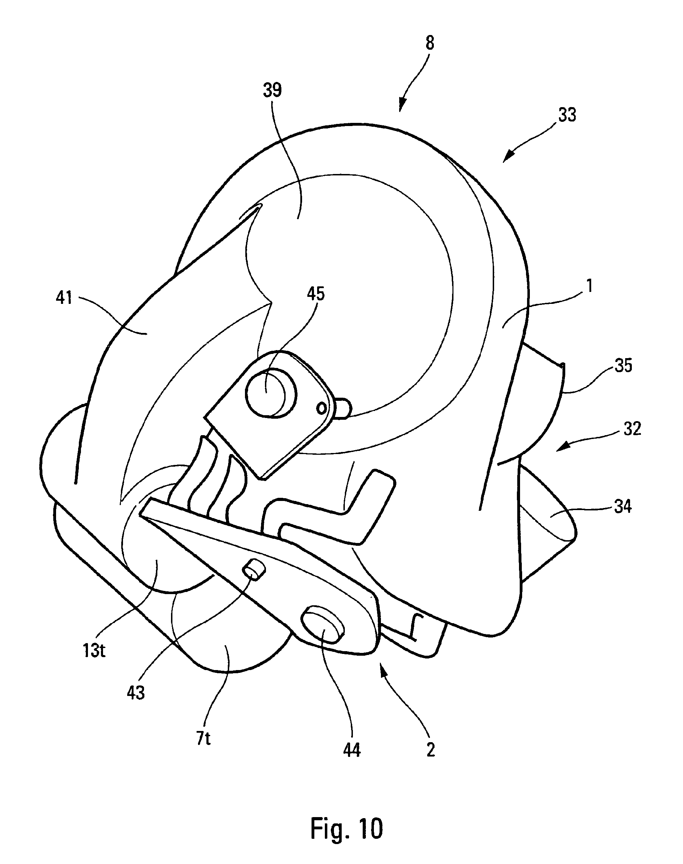

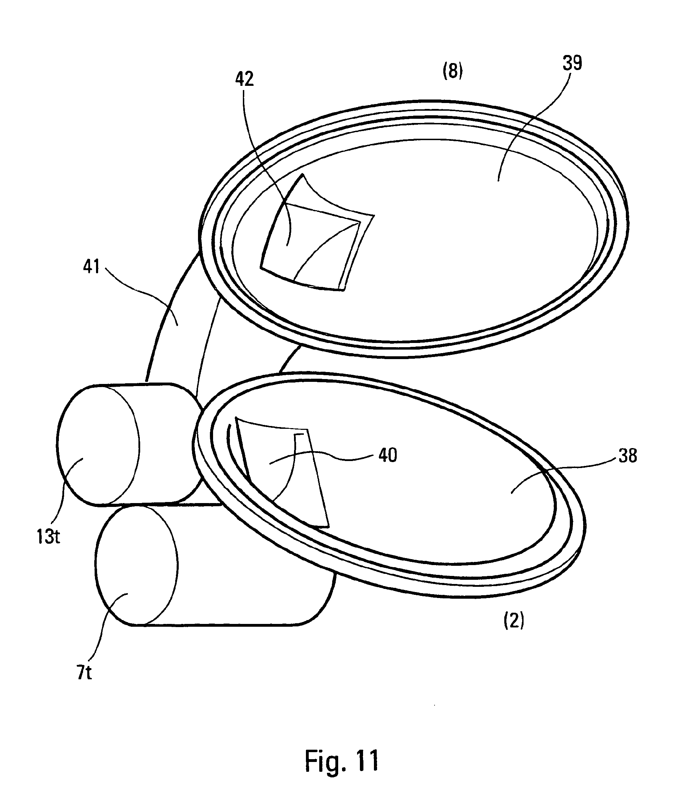

Sur la figure 10, on a représenté en perspective un mode de réalisation de l'ensemble du corps 1 et des actionneurs 7t et 1 3t se trouvant à l'intérieur de la paroi enveloppante perforée 37 et du sac souple 12. Sur cette figure, on peut voir que les actionneurs hydrauliques auxiliaire et principal 7t et 13t ont été disposés proches l'un de l'autre, au voisinage du ventricule gauche artificiel 2. De plus, comme le représente plus clairement la figure 11, les parois extérieures 2E et 8E des chambres à fluide hydraulique 5 et 11 des ventricules artificiels 2 et 8 (voir également la figure 4) sont formées par des couvercles amovibles 38 et 39 venant obturer lesdits ventricules, respectivement.

L'actionneur auxiliaire 7t communique avec la chambre 5 du ventricule gauche artificiel 2 par un orifice 40 traversant le couvercle 38 et faisant office de la liaison 15 de la figure 5. L'actionneur principal 13t communique, d'une part, avec la chambre 5 du ventricule artificiel 2 par le même orifice 40 et, d'autre part, avec la chambre 11 du ventricule artificiel 8 par l'intermédiaire d'un conduit extérieur 41 débouchant par un orifice 42 dans le couvercle 39. Le conduit 41 et les orifices 40 et 42 correspondent à la liaison 16 de la figure 5.

Comme représenté sur la figure 11, les actionneurs 7t et 13t, les couvercles 38 et 39 et le conduit 41 peuvent être solidaires les uns des autres pour former une unité de construction.

Sur la figure 10, on a représenté de plus des éléments électroniques de pilotage, tels qu'un capteur 43 de la pression à l'intérieur du sac souple 12, un capteur 44 de la pression dans le ventricule artificiel gauche 2 et un capteur 45 de la pression dans le ventricule artificiel droit 8.

## Revendications

1. Prothèse cardiaque implantable dans la cavité péricardique d'un patient, ladite prothèse étant apte à remplacer les ventricules gauche et droit naturels dudit patient après ablation de ceux-ci et comportant :
- un corps rigide (1) dans lequel sont agencés des ventricules artificiels gauche et droit (2, 8), chacun de ces ventricules artificiels comprenant une membrane souple pulsatile (3, 9) :
■ qui est apte à battre sous l'action d'un fluide hydraulique, et
■ qui est disposée dans une cavité que ladite membrane partage de façon étanche en deux chambres (4, 5 - 10, 11) dont l'une est destinée à la circulation du sang et dont l'autre est destinée audit fluide hydraulique, la chambre à sang (4) du ventricule artificiel gauche (2) étant destinée à être reliée à l'oreillette naturelle gauche (OG) et à l'aorte (AO), alors que la chambre à sang (10) du ventricule artificiel droit (8) est destinée à être reliée à l'oreillette naturelle droite (OD) et à l'artère pulmonaire (AP) ;
- deux actionneurs hydrauliques reliés aux chambres à fluides hydraulique (5, 11) desdits ventricules artificiels (2), pour alternativement y injecter et en expulser du fluide hydraulique et assurer des valeurs désirées de débits diastoliques et systoliques ; et
- un sac souple (12) entourant, de façon ample et étanche, au moins une partie dudit corps rigide en enfermant lesdits actionneurs hydrauliques, ledit sac souple servant à la fois de réservoir de fluide hydraulique (14) pour lesdits actionneurs hydrauliques et de chambre de compliance,
**caractérisée en ce que :**
- l'un desdits actionneurs (13t) est principal et est disposé entre les chambres à fluide hydraulique (5, 11) des deux ventricules artificiels (2, 8), dont l'un (8) est menant et l'autre (2) est mené ;
- l'autre (7t) desdits actionneurs est auxiliaire et est disposé entre la chambre à fluide hydraulique (5) dudit ventricule artificiel mené (2) et ledit réservoir (14) de fluide hydraulique constitué par ledit sac souple (12) ;
- l'actionneur principal (13t) :
• adresse, audit ventricule artificiel menant (8), des débits diastolique et systolique de valeurs respectives désirées (18) pour ce ventricule artificiel menant (8), et
• adresse, audit ventricule artificiel mené (2) :
* un débit systolique (20) inverse dudit débit diastolique de valeur désirée pour ledit ventricule artificiel menant (8), et
* un débit diastolique (20) inverse dudit débit systolique de valeur désirée pour ledit ventricule artificiel menant (8) ; et
- l'actionneur auxiliaire (7t) adresse, audit ventricule artificiel mené (2), des débits systolique et diastolique de correction (21) pour corriger lesdits débits systolique et diastolique adressés par ledit actionneur principal (13t) audit ventricule artificiel mené (2) et pour communiquer respectivement aux débits systolique et diastolique corrigés des valeurs désirées (19) pour ledit ventricule artificiel mené (7).

2. Prothèse cardiaque selon la revendication 1,
**caractérisée en ce que** ledit actionneur auxiliaire (7t) est de moindre puissance que ledit actionneur principal (13t).

3. Prothèse cardiaque selon la revendication 2,
**caractérisée en ce que** ledit actionneur auxiliaire (7t) présente des dimensions inférieures à celles dudit actionneur principal (1 3t).

4. Prothèse cardiaque selon l'une des revendications 1 à 3,
**caractérisée en ce que** le ventricule artificiel menant correspond au ventricule artificiel droit (8), alors que le ventricule artificiel mené correspond au ventricule artificiel gauche.

5. Prothèse cardiaque selon l'une des revendications 1 à 4,
**caractérisée en ce que** ledit actionneur principal (13t) et ledit actionneur auxiliaire (7t) sont du type moto-pompe volumétrique.

6. Prothèse cardiaque selon l'une des revendications 1 à 5,
**caractérisée en ce que** ledit actionneur principal (13t) et ledit actionneur auxiliaire (7t) sont disposés au voisinage l'un de l'autre.

7. Prothèse cardiaque selon les revendications 2 et 6,
**caractérisée en ce que** lesdits actionneurs principal et auxiliaire (13t, 7t) sont disposés au voisinage du ventricule artificiel gauche (2) et communiquent en commun (à travers 40) avec la chambre à fluide hydraulique (5) dudit ventricule artificiel gauche (2) et **en ce que** ledit actionneur principal (13t) est relié à la chambre à fluide hydraulique (11) dudit ventricule artificiel droit (8) par un conduit (41) extérieur audit corps rigide (1).

## Claims

1. A cardiac prosthesis implantable in the pericardial cavity of a patient, said prosthesis being capable of replacing the natural left and right ventricles of said patient after ablation thereof and comprising:
- a stiff body (1) in which are arranged artificial left and right ventricles (2, 8), each of these artificial ventricles comprising a soft pulsatile membrane (3, 9):
• which is capable of beating under the action of a hydraulic fluid, and
• which is provided in a cavity sealingly partitioned by said membrane into two chambers (4,5-10, 11), one of which is intended for blood flow and the other is intended for said hydraulic fluid, the blood chamber (4) of the artificial left ventricle (2) being intending to be connected to the natural left atrium (OG) and to the aorta (AO), whereas the blood flow (10) of the artificial right ventricle (8) is intended to be connected to the natural right atrium (OD) and to the pulmonary artery (AP) ;
- two hydraulic actuators connected to the hydraulic fluid chambers (5, 11) of said artificial ventricles (2), for alternately injecting therein and expulsing therefrom hydraulic fluid and ensuring desired values of diastolic and systolic flow rates; and
- a soft bag (12) widely and sealingly surrounding at least one portion of said stiff body by enclosing said hydraulic actuators, said soft bag acting both as a hydraulic fluid reservoir (14) for said hydraulic actuators and as a compliance chamber,
**characterised in that**:
- one of said actuators (13t) is a main one and is provided between the hydraulic fluid chambers (5, 11) of both artificial ventricles (2, 8), one of which (8) is driving and the other (2) is driven;
- the other (7t) of said actuators is a auxiliary one and is provided between the hydraulic fluid chamber (5) of said driven artificial ventricle (2) and said reservoir (14) of hydraulic fluid made up of said soft bag (12);
- the main actuator (13t):
• transmits to said driving artificial ventricle (8) diastolic and systolic flow rates having desired respective values (18) for this driving artificial ventricle (8), and
• transmits to said driven artificial ventricle (2):
* a systolic flow rate (20) opposite to said diastolic flow rate of a desired value for said driving artificial ventricle (8), and
* a diastolic flow rate (20) opposite to said systolic flow rate of a desired value for said driving artificial ventricle (8); and
- the auxiliary actuator (7t) transmits to said driven artificial ventricle (2) correction systolic and diastolic flow rates (21) for correcting said systolic and diastolic flow rates transmitted by said main actuator (13t) to said driven artificial ventricle (2) and for communicating respectively to the corrected systolic and diastolic flow rates desired values (19) for said driven artificial ventricle (7).

2. The cardiac prosthesis according to claim 1,
**characterised in that** said auxiliary actuator (7t) has a lesser power than said main actuator (13t).

3. The cardiac prosthesis according to claim 2,
**characterised in that** said auxiliary actuator (7t) has a smaller size than said main actuator (13t).

4. The cardiac prosthesis according to one of claims 1 to 3,
**characterised in that** the driving artificial ventricle corresponds to the right artificial ventricle (8), whereas the driven artificial ventricle corresponds to the left artificial ventricle.

5. The cardiac prosthesis according to one of claims 1 to 4,
**characterised in that** said main actuator (13t) and said auxiliary actuator (7t) are of the volumetric motor pump type.

6. The cardiac prosthesis according to one of claims 1 to 5,
**characterised in that** said main actuator (13t) and said auxiliary actuator (7t) are provided in the vicinity of each other.

7. The cardiac prosthesis according to claims 2 and 6,
**characterised in that** said main and auxiliary actuators (13t, 7t) are provided in the vicinity of the left artificial ventricle (2) and communicate commonly (through 40) with the hydraulic fluid chamber (5) of said left artificial ventricle (2) and **in that** said main actuator (13t) is connected to the hydraulic fluid chamber (11) of said right artificial ventricle (8) through a duct (41) outside said stiff body (1).

## Patentansprüche

1. Herzprothese, implantierbar in der Perikardhöhle eines Patienten, wobei die Prothese dazu geeignet ist, den natürlichen linken und rechten Ventrikel des Patienten nach der Entfernung dieser zu ersetzen und umfassend:
- einen starren Körper (1), in dem der linke und der rechte künstliche Ventrikel (2, 8) angeordnet sind, wobei jeder dieser künstlichen Ventrikel eine weiche, pulsierende Membran (3, 9) umfasst:
· die dazu in der Lage ist, unter der Einwirkung einer hydraulischen Flüssigkeit zu pulsieren, und
· die in einem Hohlraum angeordnet ist, den die Membran auf dichte Weise in zwei Kammern 4, 5 - 10, 11) teilt, wobei die erste für den Blutkreislauf bestimmt ist, und wobei die andere für die hydraulische Flüssigkeit bestimmt ist, wobei die Blutkammer (4) des linken künstlichen Ventrikels (2) dazu bestimmt ist, mit dem linken natürlichen Vorhof (OG) und mit der Aorta (AO) verbunden zu sein, während die Blutkammer (10) des rechten künstlichen Ventrikels (8) dazu bestimmt ist, mit dem rechten natürlichen Vorhof (OD) und mit der Lungenarterie (AP) verbunden zu sein.
- zwei hydraulische Antriebe, die mit den Kammern mit hydraulischen Flüssigkeiten (5, 11) der künstlichen Ventrikel (2) verbunden sind, um abwechselnd in diese hydraulische Flüssigkeit zu injizieren und daraus auszustoßen und die gewünschten Werte der diastolischen und systolischen Leistung sicherzustellen; und
- einen weichen Beutel (12), der auf weiträumige und dicht Weise zumindest einen Teil des starren Köpers umgibt, indem er die hydraulischen Antriebe einschließt, wobei der weiche Beutel gleichzeitig als Behälter für hydraulische Flüssigkeit (14) für die hydraulischen Antriebe und als Compliance-Kammer dient,
**dadurch gekennzeichnet, dass**:
einer der Antriebe (13t) der Hauptantrieb ist und zwischen den Kammern mit hydraulischer Flüssigkeit (5, 11) der zwei künstlichen Ventrikel (2, 8) angebracht ist, wobei der eine (8) ableitend und der andere (2) aufnehmend ist;
- der andere (7t) der Antriebe ein Zusatzantrieb ist und zwischen der Kammer mit hydraulischer Flüssigkeit (5) des aufnehmenden Ventrikels (2) und dem Behälter (14) von hydraulischer Flüssigkeit, gebildet von dem weichen Beutel (12), angebracht ist;
- der Hauptantrieb (13t):
· an den ableitenden künstlichen Ventrikel (8) diastolische und systolische Leistungen mit entsprechenden gewünschten Werten (18) für diesen ableitenden künstlichen Ventrikel (8) richtet, und
· und an das aufnehmende künstliche Ventrikel (2) Folgendes richtet:
* seine systolische Leistung (20), die der diastolischen Leistung entgegengesetzt ist, mit einem gewünschten Wert für den ableitenden künstlichen Ventrikel (8), und
* seine diastolische Leistung (20), die der systolischen Leistung entgegengesetzt ist, mit einem gewünschten Wert für den ableitenden künstlichen Ventrikel (8); und
- der Zusatzantrieb (7t) an den aufnehmenden künstlichen Ventrikel (2) systolische und diastolische Korrekturleistungen (21) richtet, um die systolische und diastolische Leistung zu korrigieren, die vom Hauptantrieb (13t) an den aufnehmenden künstlichen Ventrikel gerichtet sind, und um jeweils an die korrigierte systolische und diastolische Leistung gewünscht Werte (19) für den aufnehmenden künstlichen Ventrikel zu kommunizieren.

2. Herzprothese nach Anspruch 1, **dadurch gekennzeichnet, dass** der Zusatzantrieb (7t) eine geringere Leistung als der Hauptantrieb aufweist.

3. Herzprothese nach Anspruch 2, **dadurch gekennzeichnet, dass** der Zusatzantrieb (7t) Abmessungen aufweist, die geringer als diejenigen des Hauptantriebs sind.

4. Herzprothese nach einem der Ansprüche 1 bis 3, **dadurch gekennzeichnet, dass** der ableitende künstliche Ventrikel dem rechten künstliche Ventrikel (8) entspricht, während der aufnehmende künstliche Ventrikel dem linken künstlichen Ventrikel entspricht.

5. Herzprothese nach einem der Ansprüche 1 bis 4, **dadurch gekennzeichnet, dass** der Hauptantrieb (13t) und der Zusatzantrieb (7t) des Typs volumetrische Motorpumpe sind.

6. Herzprothese nach einem der Ansprüche 1 bis 5, **dadurch gekennzeichnet, dass** der Hauptantrieb (13t) und der Zusatzantrieb (7t) einander benachbart angebracht sind.

7. Herzprothese nach Anspruch 2 und 6, **dadurch gekennzeichnet, dass** der Haupt- und der Zusatzantrieb (13t, 7t) in der Nähe des linken künstlichen Ventrikels (2) angebracht sind und zusammen (über 40) mit der Kammer mit hydraulischer Flüssigkeit (5) des linken künstlichen Ventrikels (2) kommunizieren, und **dadurch**, dass der Hauptantrieb (13t) mit der Kammer mit hydraulischer Flüssigkeit (11) des rechten künstlichen Ventrikels (8) durch eine Röhre (41) außerhalb des starren Körpers (1) verbunden ist.
